# EUROPÄISCHE PATENTSCHRIFT

(11) **EP 1 551 977 B1**
(45) Veröffentlichungstag und Bekanntmachung des Hinweises auf die Patenterteilung: **27.04.2011**
(21) Anmeldenummer: 03753550.7
(22) Anmeldetag: 14.10.2003
(51) Int. Cl.: C12N 15/62, C07K 14/525, C07K 14/705, A61K 47/48, C12N 1/19, C12N 5/10

(54) **SELEKTIVE, LOKALE AKTIVIERUNG VON MITGLIEDERN DER TNF-LIGANDEN FAMILIE VON SYSTEMISCH INAKTIVEN NICHT-ANTIKÖRPER-TNF-LIGANDEN-FUSIONSPROTEINEN**
SELECTIVE, LOCAL ACTIVATION OF MEMBERS OF THE TNF LIGAND FAMILY OF SYSTEMICALLY INACTIVE NON-ANTIBODY TNF LIGAND FUSION PROTEINS
ACTIVATION LOCALE SELECTIVE DE MEMBRES DE LA FAMILLE DES LIGANDS TNF PAR DES PROTEINES HYBRIDES DE LIGANDS TNF NON-ANTICORPS SYSTEMIQUEMENT INACTIVES

(30) Priorität: 14.10.2002 DE 10247755
(43) Veröffentlichungstag der Anmeldung: 13.07.2005
(73) Patentinhaber: Pfizenmaier, Klaus, 75233 Tiefenbronn (DE); Wajant, Harald, 97270 Kist (DE)
(72) Erfinder: Pfizenmaier, Klaus, 75233 Tiefenbronn (DE); Wajant, Harald, 97270 Kist (DE)
(74) Vertreter: Graf von Stosch, Andreas
(86) Internationale Anmeldenummer: PCT/EP2003/011357
(87) Internationale Veröffentlichungsnummer: WO 2004/035794

(56) Entgegenhaltungen:
- WO-A-02/22680
- WO-A-02/22833
- XIANG J: "TARGETING CYTOKINES TO TUMORS TO INDUCE ACTIVE ANTITUMOR IMMUNE RESPONSES BY RECOMBINANT FUSION PROTEINS" HUMAN ANTIBODIES, AMSTERDAM, NL, Bd. 9, 1996, Seiten 23-36, XP000940526 ISSN: 1093-2607
- WAJANT H ET AL: "DIFFERENTIAL ACTIVATION OF TRAIL-R1 AND -2 BY SOLUBLE AND MEMBRANE TRAIL ALLOWS SELECTIVE SURFACE ANTIGEN-DIRECTED ACTIVATION OF TRAIL-R2 BY A SOLUBLE TRAIL DERIVATIVE" ONCOGENE, BASINGSTOKE, HANTS, GB, Bd. 20, Nr. 30, 2001, Seiten 4101-4106, XP001062620 ISSN: 0950-9232

## Beschreibung

Die vorliegende Erfindung betrifft Polypeptide, bestehend aus einer Effektor- sowie einer Zelloberflächenmolekül-Bindungsdomäne, die durch einen Peptidlinker verknüpft sind. Die Effektordomäne ist ein Fragment, insbesondere die extrazelluläre Domäne, eines Mitglieds der TNF-Ligandenfamilie (Modul 1), welches als solches biologisch inaktiv oder eingeschränkt aktiv ist. Die Zelloberflächenmolekül-Bindungsdomäne (Modul 2) ist ein Aminosäureabschnitt, der selektiv an eine Oberflächenstruktur der Plasmamembran, vorzugsweise an ein Membranprotein, bindet, jedoch nicht von einem Immunglobulin abgeleitet ist. Des Weiteren stellt die Erfindung für die Polypeptide codierende Nukleinsäurekonstrukte, diese enthaltende Vektoren, damit transfizierte Wirtszellen, die genannten Erfindungsgegenstände enthaltende pharmazeutische Zusammensetzungen, Verfahren zur Herstellung der erfindungsgemäßen Polypeptide sowie Verwendungen erfindungsgemäßer Gegenstände zu therapeutischen Zwecken bereit.

Zytokine, bspw. Mitglieder der TNF-Ligandenfamilie, bspw. TRAIL (TNF Related Apoptosis Inducing Ligand), auch Apo2L genannt (Wiley et al. (1995), Immunity 6: 673-682, Pitti et al. (1996) J Biol Chem 271: 12687-12689), und bspw. FasL zeigen in *in vitro* Untersuchungen eine starke apoptotische Wirkung auf viele Tumorzellen tierischen und menschlichen Ursprungs. Im Falle von TRAIL scheint es so zu sein, dass maligne Zellen nicht beeinträchtigt werden. In den untersuchten präklinischen Tiermodellen (Maus, Affe) wurden darüber hinaus keinerlei Anhaltspunkte für eine akute Toxizität oder andere systemische Nebenwirkungen von TRAIL, die als therapiebegrenzend anzusehen wären, festgestellt (Walczak et al. (1999) Nat Med 5:157-163, Ashkenazi et al. (1999) J Clin Invest 104: 155-162). Neuere in vitro Untersuchungen an primären humanen Hepatozyten zeigten allerdings eine starke zytotoxische Wirkung am Beispiel eines rekombinant hergestellten TRAIL-Produktes bzw. von membranständigem TRAIL der natürlich vorkommenden Form dieses Zytokins (Jo et al. (2000) Nat Med 6: 564-567, Ichikawa et al. (2001) Nat Med 7: 954-960). Damit wird zur Zeit eine klinische systemische Anwendung von speziellen löslichen Varianten von Mitgliedern der TNF-Ligandenfamilie (insbesondere TNF, FasL, TRAIL, CD40L), welche bioaktiv sind und daher im Wesentlichen die Wirkung des membranständigen Liganden haben, aufgrund der auftretenden Nebenwirkungen ausgeschlossen. Gleiches gilt für agonistische Antikörper, die die zu diesen Liganden korrespondierenden Rezeptoren aktivieren. So wurde z.B. für den FasL (Ligand des Fas-Rezeptors (Fas, CD95)), dem Prototyp apoptotischer Zytokine, eine klinische Anwendung *a priori* aus Sicherheitsgründen unterlassen, da agonistische Antikörper gegen seinen Rezeptor, Fas, *in vivo* extrem hepatotoxisch sind (Ogasawara et al.(1993) Nature 364: 806-809). Schließlich wurde auch gezeigt, dass FasL in löslicher Form praktisch im Unterschied zu seiner membranständigen Form keine Bioaktivität besitzt (Schneider et al. (1998) J. Exp. Med. 187: 1205-1213).

Damit sind die nach dem Stand der Technik verfügbaren Varianten der Mitglieder der TNF-Ligandenfamilie entweder auf Grund mangelnder Bioaktivität oder extremer Nebenwirkungen zur therapeutischen Anwendung, bspw. zur Behandlung von Tumoren, nicht oder nur sehr begrenzt (z.B. im Falle von TNF unter sog. "isolated limb perfusion" Bedingungen) einsetzbar.

Die WO 02/22680 offenbart Fusionsproteine, bei denen an sich biologisch nicht aktive bzw. eingeschränkt aktive Fragmente von bspw. TNF-Zytokinen über einen Peptidlinker mit einem Antigen-bindenden Antikörper bzw. einem antigenbindenden Fragment davon verbunden sind. Es besteht jedoch ein Bedarf an alternativen Systemen, da nicht selten für bestimmte Zelloberflächenmoleküle keine entsprechenden Antikörper (oder Antikörperfragmente) verfügbar sind oder die Antigenbindung nicht wie erwartet, bspw. nicht mit der entsprechenden Spezifität, erfolgt.

Der vorliegenden Erfindung liegt nunmehr die Aufgabe zugrunde, ein alternatives, insbesondere von Antikörpern unabhängiges System bereitzustellen, mit dem es gelingt, die Wirkung von Liganden der TNF-Familie (bzw. Fragmenten davon) gerichtet und gewebs- bzw. zellspezifisch zu entfalten und damit unerwünschte, u.U. systemische Nebenwirkungen auf nicht zum Zielgewebe gehörigen Geweben/Zellen bei einer klinischen Anwendung zu vermeiden oder zumindest stark einzuschränken.

Diese Aufgabe wird durch die in den Ansprüchen gekennzeichneten Ausführungsformen der vorliegenden Erfindung gelöst.

Es wird erfindungsgemäß ein Polypeptid bereitgestellt, umfassend einen Abschnitt (1), der eine Zelloberflächenmolekül-Bindungsdomäne (auch sog. Modul 2) enthält, wobei die Zelloberflächenmolekül-Bindungsdomäne nicht von einem Immunglobulin abgeleitet ist, einen Abschnitt (2), der ein Peptidlinker ist, und einen Abschnitt (3), der ein als solches biologisch inaktives/eingeschränkt aktives Fragment eines Mitglieds der TNF-Ligandenfamilie (im folgenden auch "TNF-Zytokine" oder "TNF-Rezeptor-Ligandenfamilie" genannt) (auch sog. Modul 1) enthält, wobei das Fragment in Abschnitt (3) eine extrazelluläre Domäne eines Mitglieds der TNF-Ligandenfamilie oder eine mindestens 80%ige sequenzhomologe Variante der nativen Sequenz der extrazellulären Domäne aufweist, und wobei das Fragment in Abschnitt 3 nur durch Bindung des Abschnitts (1) an das Zelloberflächenmolekül biologisch vollständig aktiv wird.

Gemäß einer bevorzugten Ausführungsform sind die Abschnitte (1) bis (3) im Polypeptid (= erfindungsgemäßes Fusionsprotein = erfindungsgemäßes Proteinkonstrukt) von N- nach C-terminal angeordnet.

Der vorliegenden Erfindung die Feststellung zugrunde, dass natürlicherweise membranständig vorkommende Liganden der TNF-Familie, wenn sie vom Organismus in eine lösliche, der extrazellulären Domäne entsprechende Form proteolytisch prozessiert werden, entweder biologisch gänzlich inaktiv oder nur noch eingeschränkt, z.B auf bestimmten Membranrezeptor-Subtypen, wirksam sind. Dies gilt auch für rekombinant hergestellte Derivate, die der extrazellulären Domäne des jeweiligen Liganden entsprechen. Interessanterweise sind solche biologisch unwirksamen, löslichen Liganden durchaus noch in der Lage, an ihren komplementären Membranrezeptor zu binden, ohne jedoch eine Aktivierung des Rezeptors, d.h. Signaltransduktion und Auslösung einer zellulären Reaktion, zu bewirken. Erfindungsgemäße Erkenntnis ist es, dass ein derart inaktiver/eingeschränkt aktiver Ligand (Modul 1) durch Fusion mit einer weiteren Peptidkomponente (Modul 2), die nicht aus einem Antikörper bzw. Antikörperfragment besteht und die eine vom TNF-Liganden-Anteil unabhängige spezifische Bindung an eine zellmembranständige Struktur erlaubt, wieder (volle) biologische Wirksamkeit erlangt, wenn diese Peptidkomponente zur Bindung an eine Zeiloberfläche führt. Die so erlangte Rekonstitution der biologischen Wirkung des Moduls 1 wird sowohl gegenüber den durch Modul 2 erkannten Zielzellen selbst wirksam wie auch gegenüber benachbart liegenden Zellen, sofern diese jeweils die entsprechenden Rezeptoren für das im Fusionsprotein enthaltene TNF-LigandenFragment (Modul 1) exprimieren.

Die Zelloberflächenmolekül-Bindungsdomäne (Abschnitt (1) bzw. Modul (2)) der erfindungsgemäßen Fusionsproteine vermittelt die Bindung des Fusionsproteins an Zellen, welche die entsprechende Oberflächenstruktur aufweisen. Die Erfindung zeichnet sich aufgrund der spezifischen Module durch zwei Eigenschaften aus, die dazu führen, dass das Gesamtprotein Eigenschaften erhält, die keine der verwendeten Module allein besitzt und die nicht vorhersehbar sind.

Die erste diesbezügliche Eigenschaft ist, dass die Zelloberflächenmolekül-Bindungsdomäne im Fusionsprotein nicht durch das dem Stand der Technik entsprechende Prinzip der Verwendung von Antikörpern oder daraus abgeleiteten Fragmenten (z.B. scFv) erreicht wird, sondern dass angesichts der üblicherweise verwendeten antigenbindenden Domänen von Antikörpern unerwarteterweise andere Zelloberflächenmolekül-Bindungsdomänen dazu in der Lage sind, an sich biologisch inaktiven/eingeschränkt aktiven Fragmenten von TNF-Zytokinen durch Bindung an bestimmte Zelloberflächenstrukturen, insbesondere an Membranproteine, eine spezifisch gegen die Zielzellen gerichtete, vollständige und damit hoch wirksame Effektorwirkung zu verleihen. Bevorzugte Nicht-Immunglobulin-Domänen zur Bindung an Zelloberflächenstrukturen sind bspw. Peptidhormone oder Zytokine, kurze Peptide, aber genauso Rezeptoren für membranständige Liganden (bzw. vorzugsweise jeweils diejenigen Fragmente davon, die eine spezifische Bindung eingehen).

Die zweite Eigenschaft besteht darin, dass die Effektordomäne als solche und auch im Fusionsprotein auf Zellen, welche die Oberflächenstruktur, die durch die Zelloberflächenmolekül-Bindungsdomäne erkannt wird, nicht besitzen, biologisch inaktiv oder wenig aktiv ist, d.h. der zur Effektordomäne korrespondierende Rezeptor wird nicht aktiviert. Die überraschende, nur dem Gesamt-Fusionsprotein zukommende Eigenschaft ist, dass die Effektordomäne (an sich biologisch inaktives/eingeschränktes Fragment eines TNF-Zytokins) des Fusionsprotein nur nach Bindung an Zellen, welche die Oberflächenstruktur exprimieren, die durch die Zellobertlächenmolekül-Bindungsdomäne erkannt wird, biologisch aktiv wird. Somit aktiviert die Effektordomäne des erfindungsgemäßen Polypeptids nur den zu ihr korrespondierenden Rezeptor auf einer Zelle, welche die dem Zelloberflächenmolekül-Bindungsmodul entsprechende Oberflächenstruktur aufweist, oder auf einer Zelle, welche einer mit der Oberflächenstruktur ausgestatteten Zelle benachbart ist. Das Fusionsprotein, nicht jedoch dessen einzelne Komponenten (Modul 1 bzw. Modul 2) und auch nicht eine aktive Form der Effektordomäne, aktiviert somit den zur Effektordomäne korrespondierenden Rezeptor selektiv auf Zellen (bzw. zu diesen benachbarten Zellen), welche die Oberflächenstruktur exprimieren, die durch die Zelloberflächenmolekül-Bindungsdomäne erkannt wird. Diese neue Eigenschaft (selektive lokale Rezeptoraktivierung) des erfindungsgemäßen Polypeptids macht es daher möglich, die systemischen Nebenwirkungen zu vermeiden, die das aktive TNF-Zytokin, von dem die erfindungsgemäße Effektordomäne abgeleitet wurde, oder im Stand der Technik bekannte agonistische Antikörper, die den korrespondierenden Rezeptor aktivieren, aufweisen. Das erfindungsgemäße Polypeptid erlaubt damit eine gezielte lokale Wirkungsentfaltung im Gesamtorganismus.

Die Zelloberflächenmolekül-Bindungsdomäne wird vorteilhafter Weise derart gewählt, dass die Oberflächenstruktur, die durch diese erkannt wird, auf den Zellen, Orten oder Organen, auf denen die Effektordomäne wirksam werden soll, selektiv oder zumindest angereichert vorhanden ist. Die Zelloberflächenmolekül-Bindungsdomäne wird des Weiteren derart ausgewählt, dass sie keine Autoaktivierung des Fusionsprotein, z.B. durch Aggregation, erlaubt.

Mitglieder der TNF-Ligandenfamilie, bspw. TNF (Tumor Nekrose Faktor; GenBank Zugriffsnr. NM_000594), bspw. TRAIL (TNF Related Apoptosis Inducing Ligand; GenBank Zugriffsnr. U37518), auch Apo2L genannt, bspw. CD40L (GenBank Zugriffsnr. NM_011616) und bspw. FasL (GenBank Zugriffsnr. U11821) haben apoptotische und/oder das Immunsystem-regulierende Wirkung. Von wenigen Ausnahmen abgesehen handelt es sich bei den Mitgliedern der TNF-Ligandenfamilie um Membranproteine des Typs II. Von diesen membranständigen Formen können sich durch spezifische Proteolyse oder alternatives Spleißen jedoch in vielen Fällen auch lösliche Formen ableiten. Insbesondere können lösliche Formen auch durch gentechnische Methoden hergestellt werden. Lösliche wie auch membranständige Mitglieder der TNF-Ligandenfamilie entfalten dabei ihre biologische Wirkung durch Interaktion mit den Mitgliedern einer korrespondierenden Familie von Rezeptoren - den Mitgliedern der TNF-Rezeptorfamilie. In manchen Fällen können sich die löslichen und membranständigen Formen von Mitgliedern der TNF-Ligandenfamilie beträchtlich in ihrer Bioaktivität unterscheiden. So ist z.B lösliches TNF ein sehr starker Aktivator des TNF-R1, hat am TNF-R2 jedoch keine oder nur eine sehr eingeschränkte Wirkung. Die membranständige Form desselben Liganden kann hingegen beide TNF-Rezeptoren gleichermaßen gut aktivieren. Weitere Beispiele, in denen sich lösliche und membranständige Formen von Mitgliedern der TNF-Rezeptor-Ligandenfamilie unterscheiden, sind u.a. der FasL, der CD40L und TRAIL.

Unter einer Variante werden erfindungsgemäß allgemein Sequenzen verstanden, die mindestens 80 % der nativen Sequenz aufweisen und sich durch bspw. Deletion(en), Insertion(en) und/oder mindestens eine Mutation von der nativen Sequenz unter scheiden. Hierbei ist eine Sequenzhomologie von mindestens 90%, vorzugsweise mindestens 95% und am stärksten bevorzugt mindestens 97 % mit der entsprechenden nativen Sequenz bevorzugt. Bei einem funktionellen Fragment kann es sich um N-Terminal, C-terminal oder intrasequentiell verkürzte Sequenzen der löslichen Formen der Mitglieder der TNF-Rezeptor-Ligandenfamilie handeln. Auch biologisch aktive Varianten dieser Fragmente werden erfindungsgemäß mitoffenbart. Bei den abgeleiteten Varianten i.S. der Erfindung werden vorzugsweise selektive Rezeptorbindungseigenschaften gegeben sein, wobei die Variante z.B. hinsichtlich ihrer spezifischen Bioaktivität oder anderer Eigenschaften (Stabilität) optimiert sein kann.

Das Wirkprinzip erfindungsgemäßer Konstrukte ist insbesondere für all diejenigen Mitglieder der TNF-Rezeptor-Ligandenfamilie zutreffend, die für bestimmte Rezeptoren ausschließlich oder in besonders gutem Maße als Membranmolekül wirksam sind. Hierzu gehören neben FasL, CD40L auch TNF, TRAIL, 41 BB, CD30L und Ox40L. Besonders bevorzugt im Modul 1 (Abschnitt (3)) der erfindungsgemäßen Polypeptide sind daher solche Mitglieder der TNF-Rezeptor-Ligandenfamilie, die natürlicherweise nur als Membranprotein ihren komplementären Rezeptor aktivieren.

Ganz besonders bevorzugt ist als Abschnitt (3) eine lösliche extrazelluläre Domäne von TRAIL, FasL, CD40, 41 BBL, CD30L , Ox40L oder TNF.

Der Linkerabschnitt (2) zwischen den Abschnitten (1) (Zelloberflächenmolekül-Bindungsdomäne; Modul 2) und (3) (TNF-Rezeptor-Ligandenfragment; Modul 1) stellt sich bei erfindungsgemäßen Polypeptidkonstrukten bspw. als eine flexible Verbindung dar, vorzugsweise jedoch ohne die intrinsischen Trimerisierungseigenschaften des betreffenden TNF-Rezeptor-Ligandenfragments (Modul 1) negativ zu beeinflussen, wie nachstehend in den Beispielskonstrukten (A) und (B) gezeigt.

In Abschnitt (2) ist jede natürlich vorkommende oder synthetisch hergestellte Peptid-Sequenz denkbar. Grundsätzlich kann ein Linker einer nativen oder variierten (Teil)sequenz aller Organismen, vorzugsweise aus Vertebraten, insbesondere aus Säugetieren, vor allem aus dem Menschen, entsprechen. Ferner sind als Linker bspw. alle Sequenzabschnitte von Proteinen geeignet, die durch Ausbildung von Supersekundärstrukturen Trimere generieren. In jedem Fall können die Sequenzen von nativen Polypeptiden oder Fragmenten dieser nativen Polypeptide, die als Linker in Abschnitt (2) eines erfindungsgemäßen Polypeptids zum Einsatz kommen, auch in Form biologisch aktiver Varianten derselben im Sinne dieser Erfindung und nach obiger Definition auftreten.

Die Zelloberflächenmolekül-Bindungsdomäne (Abschnitt (1)) des Polypeptids der vorliegenden Erfindung kann z.B. den für die spezifische Interaktion mit dem Zelloberflächenmolekül erforderlichen Abschnitt eines Rezeptors für einen membranständigen Liganden umfassen. Bevorzugt im Rahmen der vorliegenden Erfindung sind dabei solche Polypeptide, die eine lösliche Liganden-bindende Domäne eines Rezeptors, insbesondere eines Mitglieds der TNF-Rezeptorfamilie, aufweisen. Hierbei darf der Rezeptor-Anteil nicht komplementär zu dem in Abschnitt (3) befindlichen TNF-Rezeptor-Ligandenfragment sein.

Gemäß einer weiteren bevorzugten Ausführungsform des Polypeptids umfasst der Abschnitt (1) mindestens den für die spezifische Interaktion mit dem Zelloberflächenmolekül erforderlichen Abschnitt eines Liganden eines membranständigen Rezeptors. Daher sind erfindungsgemäß lösliche Liganden oder Peptide, die zellständige Rezeptoren erkennen, geeignet. Es sind auch von Abschnitt (3) (Modul 1) verschiedene TNF-Rezeptor-Ligandenfragmente geeignet. Hierbei wird ein erfindungsgemäßes Polypeptid dann ganz besonders bevorzugt sein, wenn der Abschnitt (1) ein lösliches Ligand/Rezeptor-Fragment eines Säugetiers, insbesondere humanen Ursprungs, ist und mit definierten komplementären Zielstrukturen auf Säuger-, insbesondere menschlichen, Zellen und Geweben spezifisch interagiert.

Der Abschnitt (1) eines erfindungsgemäßen Polypeptids wird vorzugsweise Spezifität für ein im Tumorgewebe oder auf aktivierten Zellen des Immunsystems (z.B. T-Zellen, B-Zellen, Makrophagen, dendritische Zellen) selektiv bzw. dominant exprimiertes Zelloberflächen-Protein aufweisen. Beispiele für derartige selektiv bzw. predominant exprimierte Zelloberflächen-Proteine, die durch den Abschnitt (1) eines erfindungsgemäßen Polypeptids gebunden werden können, sind bei Tumorgeweben, wie der VEGFR bzw. der VEGFR/VEGF-Komplex, alle mutierten und Wildtyp-Formen der EGF-Rezeptorfamilie oder CD30, bei aktivierten T-Zellen bspw. CD40L oder der IL2-Rezeptor, in B-Zellen bspw. der Baff-R oder CD40, bei Makrophagen bspw. Membran-TNF oder der B7-Ligand und bei dendritischen Zellen z.B. gleichfalls der B7-Ligand .

Als bevorzugt für den Abschnitt (1) eines erfindungsgemäßen Polypeptids erweisen sich daher Protein- bzw. Peptid-Hormone, bspw. Wachstumsfaktoren, wie EGF, und Angiogenesefaktoren, wie VEGF, wie auch die löslichen extrazellulären Domänen von Rezeptoren wie z.B. alle Mitglieder der TNF-Rezeptor-Superfamilie, insbesondere TNF-R2, CD30 und CD40, sowie von anderen Rezeptoren, z.B. CD28.

Damit stellt sich ein bevorzugtes erfindungsgemäßes Polypeptid als ein rekombinantes Fusionsprotein dar, das prinzipiell folgende Strukturelemente (Monomer) in einer definierten Abfolge enthält: Abschnitt (1), bspw. N-terminal, ein Ligand-, Rezeptorfragment oder Peptid; Abschnitt (2) eine Linkersequenz; Abschnitt (3), bspw. C-terminal, der humanen extrazellulären Domäne des FasL oder des TNF oder des CD40L. Analog können bspw. CD30L, Ox40L oder andere Mitglieder der TNF-Familie als Abschnitt (3) in entsprechenden erfindungsgemäßen Polypeptiden dienen.

Ein weiterer Gegenstand der vorliegenden Erfindung sind Nukleinsäuren mit Nukleotidsequenzen (insbesondere DNA-Sequenzen), die für Fusionsproteine der vorgenannten erfindungsgemäßen Art codieren (Nukleinsäurekonstrukte) oder einen solchen für ein erfindungsgemäßes Polypeptid codierenden Bereich enthalten. Derartige Nukleinsäurekonstrukte werden bevorzugt in Expressionsvektoren exprimiert. Die entsprechenden Vektoren, die eine DNA-Sequenz für die erfindungsgemäßen Fusionsproteine enthalten, sind somit ebenfalls Gegenstand der vorliegenden Erfindung. Vorzugsweise weisen erfindungsgemäße Vektoren die Fähigkeit zur Expression und/oder Amplifikation in einer prokaryontischen und/oder eukaryontischen Zelle auf. Insbesondere betrifft die vorliegende Erfindung auch retrovirale Vektoren sowie auch all jene Vektorsysteme, die gentherapeutisch zur Anwendung kommen können, z.B. auch adenovirale Vektorsysteme. Im Rahmen der vorliegenden Erfindung werden damit auch gentherapeutische Verfahren mit erfindungsgemäßen Vektoren oder Nukleinsäurekonstrukten als Behandlungsmethode für die erfindungsgemäß offenbarten medizinischen Indikationen offenbart.

Ein weiterer Gegenstand der vorliegenden Erfindung sind Wirtszellen, die mit Nukleinsäuren transfiziert sind, die für die erfindungsgemäßen Fusionsproteine codieren. Ganz besonders bevorzugt sind in diesem Zusammenhang Wirtszellen, die mit erfindungsgemäßen Expressionsvektoren oder erfindungsgemäßen Nukleinsäurekonstrukten transfiziert sind, wobei die Expressionsvektoren wiederum DNA-Sequenzen enthalten, die für die erfindungsgemäßen Fusionsproteine codieren.

Ein weiterer Gegenstand der vorliegenden Erfindung sind Verfahren zur Herstellung (Expression und Isolierung) von erfindungsgemäßen Polypeptiden, wobei ein erfindungsgemäßes lsolierungsverfahren typischerweise gekennzeichnet ist durch (a) Bereitstellen eines erfindungsgemäßen Vektors oder eines Nukleinsäurekonstrukts, (b) Transfektion von Zellen mit einem gemäß Verfahrensschritt (a) erhaltenen Vektor oder Nukleinsäurekonstrukt, (c) Kultivierung der gemäß (b) transfizierten Zellen, und (d) Isolierung von unter entsprechenden Bedingungen exprimierten erfindungsgemäßen Polypeptiden aus den Wirtszellen und/oder dem Kulturüberstand. Die Expression des Fusionsproteins erfolgt hierbei typischerweise nach dem Stand der Technik in geeigneten Expressionssystemen, vorzugsweise als sezerniertes Produkt stabiler Transfektanden, z.B CHO-Zellen oder in anderen tierischen Zellen wie Cos7 oder SF9 (Insektenzellen) bzw. anderen eukaryontischen Zellsystemen, insbesondere in Hefen, z.B. *Pichia pastoris.* Vorzugsweise werden die exprimierten erfindungsgemäßen Polypeptide jeweilige zur Sekretion in dem Zellsystem geeignete Leadersequenzen aufweisen. Daher werden die zur Expression eingesetzten erfindungsgemäßen Vektoren auch codierende Abschnitte enthalten, die für eine funktionelle Leadersequenz codieren.

Erfindungsgemäße Polypeptide, ggf. aber auch Nukleinsäurekonstrukte, Vektoren oder Wirtszellen, (hier unter der Kategorie "erfindungsgemäße Substanzen" bzw. "erfindungsgemäße Gegenstände" zusammengefaßt), kommen auch als Arzneimittel oder zur Herstellung eines Arzneimittels in Betracht. Ihre Verwendung ist insbesondere dann gegeben, wenn erfindungsgemäße Substanzen nach Bindung des Fusionsproteins über dessen Modul 2 (Abschnitt (1)) an ein spezifisches, (Zell-) membranständiges Zielmolekül biologische Wirkung über den korrespondierenden Rezeptor des TNF-Rezeptor-Ligandenfragments gemäß Modul 1 (Abschnitt (3)) entfalten soll. Durch geeignete Auswahl des Moduls 2 wird die TNF-Rezeptor-Ligandenaktivität der erfindungsgemäßen Substanz auf das zu behandelnde Gewebe, bzw. den zu behandelnden Zelltyp, gerichtet, und es kann ein auf die jeweilige Indikation spezifisch abgestimmtes/optimiertes Therapeutikum hergestellt werden.

Ein erfindungsgemäßes Polypeptid wird z.B. bei Anwendung als Tumortherapeutikum, insbesondere zur Behandlung solider Tumore, aber auch lymphatischer Tumore (benigne oder maligne), nach *in vivo* Verabreichung durch die Zelloberflächenmolekül-Bindungsdomäne (Modul 2) zunächst spezifisch im Tumorareal selbst oder am reaktiven TumorstromalTumorgefäßsystem gebunden und dadurch aktiviert. Die zu dem TNF-Rezeptor-Ligandenfragment (Modul 1) korrespondierenden Rezeptoren können dann aktiviert werden und z.B. Apoptose induzieren (Modul 1 z.B. lösliches Fragment des FasL oder TRAIL) oder inflitrierende T-Zellen aktivieren (Modul 1 z.B. lösliches Fragment des CD40L). Ein erfindungsgemäßes Polypeptid wird z.B. bei Anwendung als Therapeutikum in Autoimmunerkrankungen nach *in vivo* Verabreichung durch die Zelloberflächenmolekül-Bindungsdomäne (Modul 2) zunächst an einen Oberflächenmarker von aktivierten T-Zellen (z.B. CD40L) oder aktivierten Makrophagen (z.B. Membran-TNF) gebunden und dadurch biologisch vollständig aktiv. Die zu dem TNF-Rezeptor-Ligandenfragment (Modul 1) korrespondierenden Rezeptoren können dann aktiviert werden und z.B. Apoptose induzieren (Modul 1 z.B. lösliches Fragment des FasL oder TRAIL).

Die Verwendung erfindungsgemäßer Substanzen ist aber grundsätzlich auch immer dann zur Anwendung im therapeutischen Bereich erwünscht, wenn die Aktivierung einer Signaltransduktionskette, bspw. die durch die TNF-Rezeptorfamilie ausgelösten Signalkaskaden, z.B. eine apoptotische Signalkaskade, ausgelöst werden soll. Somit kommt die Verwendung erfindungsgemäßer Substanzen bei der Behandlung bzw. zur Herstellung eines Arzneimittels zur Behandlung aller hyperproliferativer Erkrankungen in Betracht, bspw. auch zur zielgerichteten Ausschaltung von Zellen des Immunsystems bei überschießenden Immunreaktionen, bspw. bei Autoimmunerkrankungen, wie z.B. multiple Sklerose, rheumatoide Arthritis, Diabetes mellitus und TEN, oder bei fehlgeleiteten Immunreaktionen gegen Fremdantigene, wie sie z.B. bei Infektionserkrankungen (bakteriell (bspw. durch Mykobakterien), viral oder protozoologisch) auftreten können. In Betracht kommt ferner die Behandlung von Stoffwechselerkrankungen oder allgemeinen hyperinflammatorischen Zuständen, insbesondere chronischen Entzündungen, bspw. auch bei Allergien, aber auch die Behandlung von Abstoßungsreaktionen durch das Immunsystem des Patienten gegen Fremdgewebe. In den vorgenannten Fällen muß jeweils die Zelloberflächenmolekül-Bindungsdomäne (Modul 2), also der Abschnitt (1) eines erfindungsgemäßen Polypeptids, charakteristische Marker auf der Oberfläche der Zielzellen, bei denen vorzugsweise eine apoptotische Signalkaskade mit dem Ziel des Zelltods ausgelöst werden soll, erkennen. Im Falle der Behandlung nach Transplantation von Fremdgewebe werden also bspw. die körpereigenen für die Abstoßungsreaktion verantwortlichen Zellen des Immunsystems des Transplantionspatienten als Zielzellen dienen. Im Falle der Behandlung von rheumatoider Arthritis werden somit bspw. die körpereigenen aktivierten Makrophagen, die wesentlich zur Erkrankung beitragen, als Zielzellen dienen.

Erfindungsgemäße Gegenstände, wie Nukleinsäurekonstrukte, Expressionsvektoren oder Wirtszellen kommen - wie vorstehend dargelegt - gleichfalls als Arzneimittel bspw. zur Behandlung der vorgenannten Erkrankungen in Betracht. In diesem Fall werden vorzugsweise dem zu behandelnden Patienten zu transfizierende Zellen entnommen, diese *in vitro* mit erfindungsgemäßen Expressionsvektoren transfiziert, kultiviert und dann als Retransplantat in den Patienten überführt. Die Transfektion wird vorzugsweise durch erfindungsgemäße Nukleinsäurekonstrukte oder Expressionsvektoren vorgenommen, welche die Expression an einen regulierbaren Promotor koppeln. Das transfizierte Eigentransplantat kann lokal appliziert, bspw. injiziert werden - abhängig von der spezifischen Erkrankung und den spezifischen Zielzellen. Eine lokale Verabreichung ist bspw. im Fall einer Tumortherapie bevorzugt. Hierbei werden Tumorzellen dem Patienten entnommen, *in vitro* transfiziert und dann vorzugsweise direkt in den Tumor injiziert. Diese Vorgehensweise eignet sich bspw. zur Behandlung von Hauttumoren (z.B. Melanomen), Tumoren des Nervensystems (z.B. Glioblastomen), usw..

Ein weiterer Gegenstand der vorliegenden Erfindung ist eine pharmazeutische Zusammensetzung, enthaltend erfindungsgemäße Polypeptide, erfindungsgemäße Nukleinsäurekonstrukte, erfindungsgemäße Vektoren und/oder erfindungsgemäße Wirtszellen sowie pharmazeutisch unbedenkliche Hilfs-, Zusatz- und/oder Trägersubstanzen (z.B. auch Lösungsvermittler). Damit wird erfindungsgemäß eine Kombination erfindungsgemäßer Substanzen mit pharmazeutisch akzeptablen Träger-, Hilfs- und/oder Zusatzstoffen offenbart. Entsprechende Herstellungswege sind bei "Remington's Pharmaceutical Sciences" (Mack Pub. Co., Easton, PA, 1980) offenbart. Für die parenterale Verabreichung kommen als Trägerstoffe bspw. steriles Wasser, sterile Kochsalzlösungen, Polyalkylenglykole, hydriertes Naphthalen und insbesondere biokompatible Lactidpolymere, Lactid/Glycolidcopolymer oder Polyoxyethylen-/Polyoxypropylencopolymere in Betracht. Derartige erfindungsgemäße Zusammensetzungen kommen für alle oben offenbarten medizinischen Indikationen in Betracht.

Grundsätzlich werden im Rahmen der vorliegenden Erfindung für erfindungsgemäße Substanzen oder erfindungsgemäße Zusammensetzungen alle im Stand der Technik bekannten Verabreichungswege offenbart. Vorzugsweise erfolgt die Verabreichung eines Arzneimittels zur Behandlung der vorgenannten Erkrankungen oder Störungen auf dem parenteralen, d.h. bspw. subkutanen, intramuskulären oder intravenösen, oder oralen oder intranasalen Verabreichungsweg. Typischerweise werden erfindungsgemäße pharmazeutische Zusammensetzungen fest, flüssig oder aerosolartig (z. B. als Spray formuliert) sein. Dabei wird die Form der Zusammensetzung je nach Art der Konfektionierung veränderlich sein.

Zusammenfassend ist festzustellen, dass erfindungsgemäß dualspezifische Fusionsproteine mit proapoptotischen und immunmodulierenden Eigenschaften zur Verfügung gestellt werden, die lösliche inaktive bzw. eingeschränkt aktive Fragmente *a priori* membranständiger TNF-Rezeptor-Liganden enthalten, die jedoch lokal durch Bindung an Zelloberflächenstrukturen, die nicht mit dem TNF-Rezeptor-Ligandenfragment interagieren, aktiviert werden können. Die Zelloberflächenmolekül-Bindungsdomäne kann- selbst ein zweites Zytokin, ein Rezeptor für membranständige Liganden oder ein anderes Nicht-Antikörper-Peptid sein (bzw. ein Fragment davon), welches eine spezifische Bindung an Zellmembranstrukturen eingeht. Durch diese Zelloberflächenmolekül-Bindungsdomäne wird die TNF-Rezeptor-Ligandenaktivität auf das zu behandelnde Gewebe bzw. die zu behandelnden Zelltypen gerichtet, und es kann ein auf die jeweilige Indikation spezifisch abgestimmtes/optimiertes Therapeutikum hergestellt werden. Insgesamt wird somit die Selektivität der TNF-Rezeptor-Ligandenwirkung mit den vorliegenden erfindungsgemäßen Polypeptiden durch zwei Mechanismen erreicht: Einerseits über die durch die Zelloberflächenmolekül-Bindungsdomäne vermittelte Anreicherung des im nicht gebundenen Zustand inaktiven, respektive eingeschränkt aktiven Fusionsproteins und wichtiger durch dessen von der Zellobeflächenmolekülbindung, d.h. lmmobilisation, abhängigen Aktivierung.

Die vorliegende Erfindung wird durch die nachfolgenden Figuren näher erläutert.

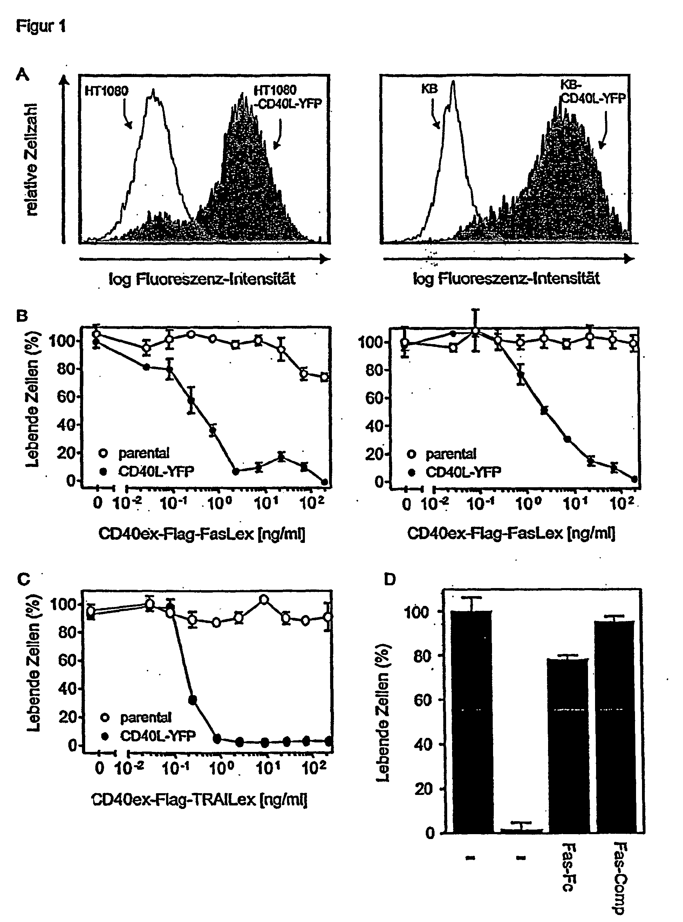

In Fig. 1 sind die Ergebnisse von Untersuchungen im Hinblick auf die präferentielle Apoptoseinduktion durch erfindungsgemäße Fusionsproteine am Beispiel CD40L-positiver Zellen dargestellt. Um die CD40L-Expression in einfacher Weise nachweisen zu können, wurde das gelb fluoreszierende Protein YFP an die intrazelluläre Domäne des CD40L fusioniert.

Fig. 1A zeigt die Expression des CD40L-YFP Fusionsproteins in parentalen KB- und HT1080-Zellen und den davon abgeleiteten Transfektanten, die stabil CD40L-YFP exprimieren, mittels FACS-Analyse. Ein entsprechendes Bild wird auch durch FACS-Analysen mit Antikörpern erhalten, welche die extrazelluläre Domäne des CD40L erkennen (Daten nicht gezeigt).

Fig. 1B bis 1D zeigen jeweils eine Auftragung der Zell-Vitalität (in %) gegen die Konzentration des jeweils angegebenen Fusionsproteins. Die eingezeichneten Kurvenverläufe geben die Ergebnisse der Behandlung von CD40L-positiven (HT1080-CD40L, KB-CD40L) oder CD40L-negativen HT1080- und KB-Zellen mit den Konstrukten CD40ex-Flag-FasLex und CD40ex-Flag-TRAILex wieder.

Bei den Experimenten gemäß Fig. 1B wurden in einer Zellkultur-Platte mit 96 Vertiefungen HT1080- und HT1080-CD40L-YFP-Zelten (linkes Diagramm) bzw. KB- und KB-CD40L-YFP-Zellen (rechtes Diagramm) über Nacht kultiviert. Am nächsten Tag wurden die Zellen mit den angegebenen Konzentrationen an CD40ex-Flag-FasLex stimuliert. Die Zellen wurden parallel zur Sensitivierung der Apoptoseinduktion mit CHX (2,5 µg/ml) behandelt. Am darauffolgenden Tag wurde die Zell-Vitalität mittels Kristall-Violett-Färbung bestimmt.

Bei den Experimenten gemäß Fig. 1C wurden in einer Zellkultur-Platte mit 96 Vertiefungen HT1080- und HT1080-CD40L-YFP-Zellen über Nacht kultiviert. Am nächsten Tag wurden die Zellen mit den angegebenen Konzentrationen an CD40ex-Flag-TRAILex stimuliert. Ansonsten wurde wie bei Fig. 1B beschrieben verfahren.

In Fig. 1D wird beispielhaft gezeigt, dass der in CD40L-exprimierenden HT1080-Zellen durch CD40ex-Flag-FasLex induzierte Zelltod durch Fas vermittelt wird. Wiederum wurden in einer Zellkultur-Platte mit 96 Vertiefungen HT1080-CD40L-YFP-Zellen über Nacht kultiviert. Am nächsten Tag wurden die Zellen mit 100 ng/ml CD40ex-Flag-FasLex stimuliert. Wo angegeben, wurden das CD40ex-Flag-FasLex-Reagenz zuvor mit 2 µg/ml Fas-Fc bzw. Fas-Comp vorinkubiert. Ansonsten wurde wie bei Fig. 1B bzw. 1C beschrieben verfahren. Durch Maskieren des FasL-Anteils in CD40ex-Flag-FasLex mit dem FasL-bindenden löslichen Reagenzien Fas-Fc und Fas-Comp. konnte die Apoptose-Induktion auf CD40L-positiven Zellen teilweise bzw. vollständig blockiert werden.

Die vorliegende Erfindung wird durch die nachfolgenden Ausführungsbeispiele näher erläutert.

### Beispiel 1

Konstruktion der erfindungsgemäßen Polypeptide CD40ex-Flag-FasLex und CD40ex-Flag-TRAILex.

### Konstrukt (A): CD40ex-Flag-FasLex

NH₂-[CD40(1-192)]-[Linker mit Flag-tag]-[FasL(139-281)]-COOH
- CD40(1-192):: extrazelluläre Domäne des humanen CD40-Rezeptors ein- schließlich Leadersequenz (AA 1-192)
- Linker1:: Linker mit Flag-Epitope (Fettdruck) GS**DYKDDDDK**EFGRGDSPGRGDSP
- FasL(139-281):: extrazelluläre Domäne des humanen FasL (AA 139-281)

### Konstrukt (B): CD40ex-Flag-TRAILex

NH₂-[CD40(1-192)]-[Linker-flag-tag]-[TRAIL(95-281)]-COOH
- CD40(1-192):: extrazelluläre Domäne des humanen CD40 Rezeptors inkl. Leadersequenz (AA 1-192)
- Linker1:: Linker mit Flag-Epitope (Fettdruck) GS**DYKDDDDK**EF
- TRAIL(95-281):: extrazelluläre Domäne des humanen TRAIL (AA 95-281)

Die Fusionsproteine wurden wie folgt hergestellt:

### CD40ex-Flag-FasLex:

1. Die der extrazellulären Domäne des CD40-Rezeptors (Aminosöuren 1-192 inkl. Leadersequenz) entsprechende cDNA wurde mittels "proof-reading"-PCR aus einem cDNA-Pool aktivierter B-Zellen amplifiziert. Dabei wurde mit Hilfe der verwendeten Primer (Nr.1160 und Nr.1161) am 5'-Ende des Amplikons eine Hind3-Schnittstelle eingefügt und am 3'-Ende eine BamH1-Schnittstelle. Das mit Hind3 und BamH1 verdaute Amplikon wurde dann in den gleichfalls mit Hind3 und BamH1 verdauten eukaryontischen Expressionsvektors pCR3 (Invitrogen) eingeführt.
2. Die der extrazellulären Domäne des FasL (Aminosäuren 139-281) entsprechende cDNA wurde mittels "proof-reading"-PCR aus einem cDNA-Pool aktivierter T-Zellen amplifiziert. Dabei wurde mit Hilfe der verwendeten Primer (Nr.1106 und Nr.1107) am 5'-Ende des Amplikons eine EcoR1-Schnittstelle eingefügt und am 3'-Ende eine Xho1-Schnittstelle. Mittels des Forward-Primers wurden des Weiteren sechs Aminosäuren als Linker eingeführt. Das mit EcoR1 und Xho1 verdaute Amplikon wurde dann in den gleichfalls mit EcoR1 und Xho1 verdaute Zwischenprodukt, das in 1. erhalten wurde, eingesetzt.
3. Abschließend wurde für analytische Zwecke zwischen die BamH1 und E-coR1-Schnittstelle des unter 2. erhaltenen Zwischenprodukts ein Flag-tag mittels Oligonukleotid-Linkern eingefügt. Das so erhaltene Konstrukt (pCR3-CD40ex-Flag-FasLex) codiert für eine Fusionsprotein mit der extrazellulären Domäne des CD40-Moleküls und der extrazellulären Domäne des Fas-Liganden, die durch einen Flag-tag sowie einigen weiteren AS verbunden sind.

### CD40ex-Flag-TRAILex:

1. Die der extrazellulären Domäne von TRAIL (Aminosäuren 95-281) entsprechende cDNA wurde mittels "proof-reading"-PCR aus einem cDNA-Pool aktivierter T-Zellen amplifiziert. Dabei wurde mit Hilfe der verwendeten Primer (Nr.1302 und Nr.1334) am 5'-Ende des Amplikons eine EcoR1-Schnittstelle eingefügt und am 3'-Ende eine Xba1-Schnittstelle. Das mit EcoR1 und Xba1 verdaute Amplikon wurde dann in den gleichfalls mit E-coR1 und Xba1 verdauten pCR3-CD40ex-Flag-FasL-ex Expressionsvektors eingeführt. Vor der Ligation wurde das durch den Verdau aus pCR3-CD40ex-Flag-FasL-ex freiwerdende FasL-Fragment entfernt. Das so erhaltene Konstrukt (pCR3-CD40ex-Flag-TRAILex) codiert für ein Fusionsprotein mit der extrazellulären Domäne des CD40 Moleküls und der extrazellulären Domäne des TRAIL-Moleküls, die durch einen Flag-tag sowie einigen weiteren Aminosäuren (AS) verbunden sind.

Zur Gewinnung von CD40ex-Flag-FasLex und CD40ex-Flag-TRAILex wurden HEK293- oder Cos7-Zellen mit den oben beschriebenen Konstrukten nach Angaben des Herstellers mit Lipofektamin (Gibco-BRL) transfiziert. 48-96 Stunden nach Transfektion wurden die Fusionsprotein-Überstände sterilfiltriert und bei 4°C bis zur weiteren Verwendung gelagert.

Alle Klonierungs- und PCR-Amplifikationsschritte erfolgten nach üblichen Standdardprozeduren mit den nachfolgenden Primern. Alle Konstrukte wurden zur Verifikation der cDNA-Sequenz sequenziert. 
Primer 1106
5' CCG GAA TTC GGC CGG GGC GAC TCA CCC GGC CGG GGC GAC TCA
CCC GAA AAA AAG GAG CTG AGG AAA GTG GCC 3'
Primer 1107
   5' CCG CTC GAG GTG CTT CTC TTA GAG CTT ATA TAA GCC G 3'
Primer 1160
   5' CCC AAG CTT CTC GCC ATG GTT CGT CTG CCT CTG CAG 3'
Primer 1161
   5' CGC GGA TCC CAG CCG ATC CTG GGG ACC ACA GAC 3' 
Primer 1302
5' CCG GAA TTC TAC GCA TAT TAC ACC TCT GAG GAA ACC ATT TCT ACA
G 3'
Primer 1334
   5' TGC TCT AGA CCA GGT CAG TTA GCC AAC TAA AAA GGC 3'

### Beispiel 2

Nachweis der CD40L-abhängigen Aktivierung von CD40ex-Flag-FasLex und CD40ex-Flag-TRAILex am Beispiel von CD40L-exprimierenden Zellen (siehe auch Abb. 1A).

CD40ex-Flag-FasLex und CD40ex-Flag-TRAILex wurde wie in Beispiel 1 beschrieben bereitgestellt. CD40L-exprimierende (HT1080-CD40L; KB-CD40L-YFP) und CD40L-negative (HT1080; KB) Zellen wurden in Zellkultur-Platten mit 96 Vertiefungen über Nacht kultiviert. Am nächsten Tag wurden die Zellen in Gegenwert von CHX (2.5 µg/ml) mit den angegebenen Konzentrationen an CD40ex-Flag-FasLex und CD40ex-Flag-TRAILex für 8 h inkubiert. Die Quantifizierung der überlebenden Zellen erfogte mittels Kristallviolett-Färbung.

### SEQUENZPROTOKOLL

### ANMELDER

| | |
|---|---|
| 1. NAME: | Prof. Dr. Klaus Pfizenmaier |
| STRASSE: | Seehausstraße 7 |
| ORT: | Tiefenbronn |
| POSTLEITZAHL: | 75233 |
| TELEFON: | 07 11/6 85 69 86 |
| TELEFAX: | 07 11/6 85 74 84 |
| | |
| 2. NAME: | PD. Dr. Harald Wajant |
| STRASSE: | Sonnenbühl 2 |
| ORT: | Leinfelden-Echterdingen |
| POSTLEITZAHL: | 70771 |
| TELEFON: | 07 11/6 85 69 86 |
| TELEFAX: | 07 11/6 85 74 46 |

| | |
|---|---|
| **BEZEICHNUNG DER ERFINDUNG**: | Selektive, lokale Aktivierung von Mitgliedern der TNF-Rezeptorfamilie durch systemisch inaktive nicht-Antikörper-TNF-Liganden-Fusionsproteine |
| **ANZAHL DER SEQUENZEN**: | 2 DNA Sequenzen |
| | 2 Proteinsequenzen |

### COMPUTERIESBARE FASSUNG:

| | |
|---|---|
| DATENTRÄGER: | Diskette |
| COMPUTER: | PC |
| BETRIEBSSYSTEM: | MS DOS |
| SOFTWARE: | Windows NT |

**Sequenzbeschreibung 1**: Codierende DNA Sequenz (unten, nur codierender DNA Strang, Nukleotid (NT) 1-1080)und translatierte Aminosäuresequenz (oben, Einzelbuchstaben Kodierung der Aminosäuren (AA) 1-359)eines erfindungsgemäßen Fusionsproteins am Beispiel von CD40ex-Flag-FasL (Konstrukt A).

1 M V R L P L Q C V L W G C L L T 16
1 ATG GTT CGT CTG CCT CTG CAG TGC GTC CTC TGG GGC TGC TTG CTG ACC 48

17 A V H P E P P T A C R E K Q Y L 32
49 GCT GTC CAT CCA GAA CCA CCC ACT GCA TGC AGA GAA AAA CAG TAC CTA 96

33 I N S Q C C S L C Q P G Q K L V 48
97 ATA AAC AGT CAG TGC TGT TCT TTG TGC CAG CCA GGA CAG AAA CTG GTG 144

49 S D C T E F T E T E C L P C G E 64
145 AGT GAC TGC ACA GAG TTC ACT GAA ACG GAA TGC CTT CCT TGC GGT GAA 192

65 S E F L D T W N R E T H C H Q H 80
193 AGC GAA TTC CTA GAC ACC TGG AAC AGA GAG ACA CAC TGC CAC CAG CAC 240

81 K Y C D P N L G L R V Q Q K G T 96
241 AAA TAC TGC GAC CCC AAC CTA GGG CTT CGG GTC CAG CAG AAG GGC ACC 288

97 S E T D T I C T C E E G W H C T 112
289 TCA GAA ACA GAC ACC ATC TGC ACC TGT GAA GAA GGC TGG CAC TGT ACG 336

113 S E A C E S C V L H R S C S P G 128
337 AGT GAG GCC TGT GAG AGC TGT GTC CTG CAC CGC TCA TGC TCG CCC GGC 384

129 F G V K Q I A T G V S D T I C E 144
385 TTT GGG GTC AAG CAG ATT GCT ACA GGG GTT TCT GAT ACC ATC TGC GAG 432

145 P C P V G F F S N V S S A F E K 160
433 CCC TGC CCA GTC GGC TTC TTC TCC AAT GTG TCA TCT GCT TTC GAA AAA 480

161 C H P W T S C E T K D L V V Q Q 176
481 TGT CAC CCT TGG ACA AGC TGT GAG ACC AAA GAC CTG GTT GTG CAA CAG 528

177 A G T N K T D V V C G P Q D R L 192
529 GCA GGC ACA AAC AAG ACT GAT GTT GTC TGT GGT CCC CAG GAT CGG CTG 576

193 G S D Y K D D D D K E F G R G D 208
577 GGA TCC GAT TAC AAA GAC GAT GAC GAT AAA GAA TTC GGC CGG GGC GAC 624

209 S P G R G D S P E K K E L R K V 224
625 TCA CCC GGC CGG GGC GAC TCA CCC GAA AAA AAG GAG CTG AGG AAA GTG 672

225 A H L T G K S N S R S M P L E W 240
673 GCC CAT TTA ACA GGC AAG TCC AAC TCA AGG TCC ATG CCT CTG GAA TGG 720

241 E D T Y G I V L L S G V K Y K K 256
721 GAA GAC ACC TAT GGA ATT GTC CTG CTT TCT GGA GTG AAG TAT AAG AAG 768

257 G G L V I N E T G L Y F V Y S K 272
769 GGT GGC CTT GTG ATC AAT GAA ACT GGG CTG TAC TTT GTA TAT TCC AAA 816

273 V Y F R G Q S C N N L P L S H K 288
817 GTA TAC TTC CGG GGT CAA TCT TGC AAC AAC CTG CCC CTG AGC CAC AAG 864

289 V Y M R N S K Y P Q D L V M M E 304
865 GTC TAC ATG AGG AAC TCT AAG TAT CCC CAG GAT CTG GTG ATG ATG GAG 912

305 G K M M S Y C T T G Q M W A R S 320
913 GGG AAG ATG ATG AGC TAC TGC ACT ACT GGG CAG ATG TGG GCC CGC AGC 960

321 S Y L G A V F N L T S A D H L Y 336
961 AGC TAC CTG GGG GCA GTG TTC AAT CTT ACC AGT GCT GAT CAT TTA TAT 1008

337 V N V S E L S L V N F E E S Q T 352
1009 GTC AAC GTA TCT GAG CTC TCT CTG GTC AAT TTT GAG GAA TCT CAG ACG 1056

353 F F G L Y K L * 359
1057 TTT TTC GGC TTA TAT AAG CTC TAA 1080

### Merkmale Konstrukt A:

Sequenz der extrazellulären Domäne von CD40 (AS 1-192)inkl. Leitsequenz: NT 1-576, AA 1-192

Sequenz des "Flag"-Epitop enthaltenden Linkers zwischen den extrazellulären Domänen von CD40 und FasL: NT 577-648, AA 193-216

Sequenz des humanen FasL Fragments (extrazelluläre Domäne, ab AA 139-281 des natürlichen, humanen FasL Moleküls): NT 649-1077, AA 217-359

### Stop Kodon: NT 1078-1080.

**Sequenzbeschreibung 2**: Codierende DNA Sequenz (unten, nur codierender DNA Strang, Nukleotid (NT) 1-1176)und translatierte Aminosäuresequenz (oben, Einzelbuchstaben Kodierung der Aminosäuren (AA) 1-391)eines erfindungsgemäßen Fusionsproteins am Beispiel von CD40ex-Flag-TRAIL (Konstrukt B).

1 M V R L P L Q C V L W G C L L T 16
1 ATG GTT CGT CTG CCT CTG CAG TGC GTC CTC TGG GGC TGC TTG CTG ACC 48

17 A V H P E P P T A C R E K Q Y L 32
49 GCT GTC CAT CCA GAA CCA CCC ACT GCA TGC AGA GAA AAA CAG TAC CTA 96

33 I N S Q C C S L C Q P G Q K L V 48
97 ATA AAC AGT CAG TGC TGT TCT TTG TGC CAG CCA GGA CAG AAA CTG GTG 144

49 S D C T E F T E T E C L P C G E 64
145 AGT GAC TGC ACA GAG TTC ACT GAA ACG GAA TGC CTT CCT TGC GGT GAA 192

65 S E F L D T W N R E T H C H Q H 80

193 AGC GAA TTC CTA GAC ACC TGG AAC AGA GAG ACA CAC TGC CAC CAG CAC 240

81 K Y C D P N L G L R V Q Q K G T 96
241 AAA TAC TGC GAC CCC AAC CTA GGG CTT CGG GTC CAG CAG AAG GGC ACC 288

97 S E T D T I C T C E E G W H C T 112
289 TCA GAA ACA GAC ACC ATC TGC ACC TGT GAA GAA GGC TGG CAC TGT ACG 336

113 S E A C E S C V L H R S C S P G 128
337 AGT GAG GCC TGT GAG AGC TGT GTC CTG CAC CGC TCA TGC TCG CCC GGC 384

129 F G V K Q I A T G V S D T I C E 144
385 TTT GGG GTC AAG CAG ATT GCT ACA GGG GTT TCT GAT ACC ATC TGC GAG 432

145 P C P V G F F S N V S S A F E K 160
433 CCC TGC CCA GTC GGC TTC TTC TCC AAT GTG TCA TCT GCT TTC GAA AAA 480

161 C H P W T S C E T K D L V V Q Q 176
481 TGT CAC CCT TGG ACA AGC TGT GAG ACC AAA GAC CTG GTT GTG CAA CAG 528

177 A G T N K T D V V C G P Q D R L 192
529 GCA GGC ACA AAC AAG ACT GAT GTT GTC TGT GGT CCC CAG GAT CGG CTG 576

193 G S D Y K D D D D K E F T S E E 208
577 GGA TCC GAT TAC AAA GAC GAT GAC GAT AAA GAA TTC ACC TCT GAG GAA 624

209 T I S T V Q E K Q Q N I S P L V 224
625 ACC ATT TCT ACA GTT CAA GAA AAG CAA CAA AAT ATT TCT CCC CTA GTG 672

225 R E R G P Q R V A A H I T G T R 240
673 AGA GAA AGA GGT CCT CAG AGA GTA GCA GCT CAC ATA ACT GGG ACC AGA 720

241 G R S N T L S S P N S K N E K A 256
721 GGA AGA AGC AAC ACA TTG TCT TCT CCA AAC TCC AAG AAT GAA AAG GCT 768

257 L G R K I N S W E S S R S G H S 272
769 CTG GGC CGC AAA ATA AAC TCC TGG GAA TCA TCA AGG AGT GGG CAT TCA 816

273 F L S N L H L R N G E L V I H E 288
817 TTC CTG AGC AAC TTG CAC TTG AGG AAT GGT GAA CTG GTC ATC CAT GAA 864

289 K G F Y Y I Y S Q T Y F R F Q E 304
865 AAA GGG TTT TAC TAC ATC TAT TCC CAA ACA TAC TTT CGA TTT CAG GAG 912

305 E I K E N T K N D K Q M V Q Y I 320
913 GAA ATA AAA GAA AAC ACA AAG AAC GAC AAA CAA ATG GTC CAA TAT ATT 960

321 Y K Y T S Y P D P I L L M K S A 336
961 TAC AAA TAC ACA AGT TAT CCT GAC CCT ATA TTG TTG ATG AAA AGT GCT 1008

337 R N S C W S K D A E Y G L Y S I 352
1009 AGA AAT AGT TGT TGG TCT AAA GAT GCA GAA TAT GGA CTC TAT TCC ATC 1056

353 Y Q G G I F E L K E N D R I F V 368
1057 TAT CAA GGG GGA ATA TTT GAG CTT AAG GAA AAT GAC AGA ATT TTT GTT 1104

369 S V T N E H L I D M D H E A S F 384
1105 TCT GTA ACA AAT GAG CAC TTG ATA GAC ATG GAC CAT GAA GCC AGT TTT 1152

385 F G A F L V G * 391
1153 TTC GGG GCC TTT TTA GTT GGC TAA 1176

### Merkmale Konstrukt B:

Sequenz der extrazellulären Domäne von CD40 (AS 1-192)inkl. Leitsequenz: NT 1-576, AA 1-192

Sequenz des "Flag"-Epitop enthaltenden Linkers zwischen den extrazellulären Domänen von CD40 und TRAIL: NT 577-612, AA 193-204

Sequenz des humanen FasL Fragments (extrazelluläre Domäne, ab AA 139-281 des natürlichen, humanen FasL Moleküls): NT 613-1173, AA 205-391

Stop Kodon: NT 1174-1176.

## Patentansprüche

1. Polypeptid, umfassend einen Abschnitt (1), der eine Zelloberflächenmolekül-Bindungsdomäne enthält, wobei die Zelloberflächenmolekül-Bindungsdomäne nicht von einem Immunglobulin abgeleitet ist, einen Abschnitt (2), der ein Peptidlinker ist, und einen Abschnitt (3), der ein als solches biologisch inaktives/eingeschränkt aktives Fragment eines Mitglieds der TNF-Ligandenfamilie enthält, wobei das Fragment in Abschnitt (3) eine extrazelluläre Domäne eines Mitglieds der TNF-Ligandenfamilie oder eine mindestens 80%ige sequenzhomologe Variante der nativen Sequenz der extrazellulären Domäne aufweist, und wobei das Fragment in Abschnitt 3 nur durch Bindung des Abschnitts (1) an das Zelloberflächenmolekül biologisch vollständig aktiv wird.

2. Polypeptid nach Anspruch 1, wobei die Abschnitte (1) bis (3) von N- nach C-terminal angeordnet sind.

3. Polypeptid nach Anspruch 2, wobei der Abschnitt (3) die extrazelluläre Domäne von TRAIL, FasL, TNF, 41 BBL, CD40L, CD30L oder Ox40L enthält.

4. Polypeptid nach einem der vorhergehenden Ansprüche, wobei die Zelloberflächenmolekül-Bindungsdomäne an ein Membranprotein bindet.

5. Polypeptid nach Anspruch 4, wobei die Zelloberflächenmolekül-Bindungsdomäne mindestens den für die spezifische Interaktion mit dem Zelloberflächenmolekül erforderlichen Abschnitt eines Liganden von einem membranständigen Rezeptor umfasst.

6. Polypeptid nach Anspruch 5, wobei der Ligand ein Rezeptor-bindendes Peptid- oder Proteinhormon oder ein Rezeptor-bindendes Fragment eines Zytokins ist.

7. Polypeptid nach Anspruch 6, wobei das Zytokin-Fragment ein Fragment eines Mitglieds der TNF-Ligandenfamilie ist, mit der Maßgabe, dass das Mitglied der TNF-Ligandenfamilie, von dem die Abschnitte (1) und (3) stammen, unterschiedlich ist.

8. Polypeptid nach Anspruch 6, wobei das Hormon aus der Gruppe, bestehend aus Wachstumsfaktoren, insbesondere EGF, und Angiogenesefaktoren, insbesondere VEGF, ausgewählt ist.

9. Polypeptid nach Anspruch 4, wobei die Zelloberflächenmolekül-Bindungsdomäne mindestens den für die spezifische Interaktion mit dem Zelloberflächenmolekül erforderlichen Abschnitt eines Rezeptors für einen membranständigen Liganden umfasst, mit der Maßgabe, dass der Rezeptor-Abschnitt nicht an die extrazelluläre Domäne des Mitglieds der TNF-Ligandenfamilie gemäß Abschnitt (3) bindet.

10. Polypeptid nach Anspruch 9, wobei der Rezeptor aus der TNF-Rezeptor-Superfamilie ausgewählt ist.

11. Polypeptid nach Anspruch 9 oder 10, wobei der Rezeptor aus der Gruppe, bestehend aus TNF-R2, CD30, CD40 und CD28, ausgewählt ist.

12. Nukleinsäurekonstrukt, enthaltend eine Nukleotidsequenz, die für ein Polypeptid nach einem der vorhergehenden Ansprüche codiert.

13. Vektor, enthaltend das Nukleinsäurekonstrukt nach Anspruch 12.

14. Wirtszelle, enthaltend das Nukleinsäurekonstrukt nach Anspruch 12 und/oder den Vektor nach Anspruch 13.

15. Verfahren zur Isolierung eines Polypeptids nach einem der Ansprüche 1 bis 11, wobei
(a) ein Vektor gemäß Anspruch 13 oder ein Nukleinsäurekonstrukt gemäß Anspruch 12 hergestellt wird,
(b) Zellen mit einem gemäß Verfahrensschritt (a) erhaltenen Vektor oder Nukleinsäurekonstrukt transfiziert werden,
(c) die gemäß (b) transfizierten Zellen kultiviert werden, und
(d) unter entsprechenden Bedingungen exprimierte Polypeptide aus den Wirtszellen und/oder dem Kulturüberstand isoliert werden.

16. Polypeptid nach einem der Ansprüche 1 bis 11, Nukleinsäurekonstrukt nach Anspruch 12, Vektor nach Anspruch 13 oder Wirtszelle nach Anspruch 14 als Arzneimittel.

17. Polypeptid nach einem der Ansprüche 1 bis 11, Nukleinsäurekonstrukt nach Anspruch 12, Vektor nach Anspruch 13 oder Wirtszelle nach Anspruch 14 zur Verwendung in der Behandlung von Krebserkrankungen, insbesondere soliden oder lymphatischen Tumoren, Infektionskrankheiten, Stoffwechselerkrankungen, Entzündungszuständen, Autoimmunerkrankungen, insbesondere rheumato/arthritische Erkrankungen.

18. Pharmazeutische Zusammensetzung, mindestens enthaltend ein Polypeptid nach einem der Ansprüche 1 bis 11 und/oder ein Nukleinsäurekonstrukt nach Anspruch 12 und/oder einen Vektor nach Anspruch 13 und/oder Wirtszellen nach Anspruch 14 sowie pharmazeutisch unbedenkliche Hilfs-, Zusatz- und/oder Trägersubstanzen.

19. Pharmazeutische Zusammensetzung nach Anspruch 18 zur Verwendung in der Behandlung von Krebserkrankungen, insbesondere soliden oder lymphatischen Tumoren, Infektionskrankheiten, Stoffwechselerkrankungen, Entzündungszuständen, Autoimmunerkrankungen, insbesondere rheumato/arthritische Erkrankungen.

## Claims

1. Polypeptide comprising a segment (1), which contains a cell surface molecule binding domain, the cell surface molecule binding domain not being derived from an immunoglobulin, a segment (2), which is a peptide linker, and a segment (3), which contains a fragment of a member of the TNF ligand family that as such is biologically inactive/of restricted activity, wherein the fragment in segment (3) contains an extracellular domain of a member of the TNF ligand family or a variant having at least 80% sequence homology of the native sequence of the extracellular domain, and wherein the fragment in segment (3) becomes biologically fully active only when segment (1) binds to the cell surface molecule.

2. Polypeptide according to Claim 1, wherein segments (1) to (3) are arranged from N-terminal to C-terminal.

3. Polypeptide according to Claim 2, wherein segment (3) contains the extracellular domain of TRAIL, FasL, TNF, 41 BBL, CD40L, CD30L or Ox40L.

4. Polypeptide according to one of the preceding claims, wherein the cell surface molecule binding domain binds to a membrane protein.

5. Polypeptide according to Claim 4, wherein the cell surface molecule binding domain comprises at least that segment of a ligand of a membrane-based receptor which is required for the specific interaction with the cell surface molecule.

6. Polypeptide according to Claim 5, wherein the ligand is a receptor-binding peptide or protein hormone or a receptor-binding fragment of a cytokine.

7. Polypeptide according to Claim 6, wherein the cytokine fragment is a fragment of a member of the TNF ligand family, with the proviso that the member of the TNF ligand family from which segments (1) and (3) are derived is different.

8. Polypeptide according to Claim 6, wherein the hormone is selected from the group consisting of growth factors, especially EGF, and angiogenesis factors, especially VEGF.

9. Polypeptide according to Claim 4, wherein the cell surface molecule binding domain comprises at least that segment of a receptor for a membrane-based ligand which is required for the specific interaction with the cell surface molecule, with the proviso that the receptor segment does not bind to the extracellular domain of the member of the TNF ligand family according to segment (3).

10. Polypeptide according to Claim 9, wherein the receptor is selected from the TNF receptor superfamily.

11. Polypeptide according to Claim 9 or 10, wherein the receptor is selected from the group consisting of TNF-R2, CD30, CD40 and CD28.

12. Nucleic acid construct containing a nucleotide sequence that codes for a polypeptide according to one of the preceding claims.

13. Vector containing the nucleic acid construct according to Claim 12.

14. Host cell containing the nucleic acid construct according to Claim 12 and/or the vector according to Claim 13.

15. Process for the isolation of a polypeptide according to one of Claims 1 to 11,
wherein
(a) a vector according to Claim 13 or a nucleic acid construct according to Claim 12 is prepared,
(b) cells are transfected with a vector or nucleic acid construct obtained according to process step (a),
(c) the cells transfected according to (b) are cultivated, and
(d) polypeptides expressed under appropriate conditions are isolated from the host cells and/or the culture supernatant.

16. Polypeptide according to one of Claims 1 to 11, a nucleic acid construct according to Claim 12, a vector according to Claim 13 or a host cell according to Claim 14 as a medicament.

17. Polypeptide according to one of Claims 1 to 11, a nucleic acid construct according to Claim 12, a vector according to Claim 13 or a host cell according to Claim 14 for use in the treatment of cancer diseases, especially solid or lymphatic tumours, infectious diseases, metabolic diseases, inflammatory states and autoimmune diseases, especially rheumatic/arthritic diseases.

18. Pharmaceutical composition containing at least a polypeptide according to one of Claims 1 to 11 and/or a nucleic acid construct according to Claim 12 and/or a vector according to Claim 13 and/or host cells according to Claim 14, and pharmaceutically acceptable auxiliary substances, additives and/or excipients.

19. Pharmaceutical composition according to Claim 18 for use in the treatment of cancer diseases, especially solid or lymphatic tumours, infectious diseases, metabolic diseases, inflammatory states and autoimmune diseases, especially rheumatic/arthritic diseases.

## Revendications

1. Polypeptide, comprenant une section (1), qui contient un domaine de liaison de molécule de surface cellulaire, sachant que le domaine de liaison de molécule de surface cellulaire n'est pas dérivé d'une immunoglobuline, une section (2), qui est un peptide de liaison, et une section (3), qui contient un fragment d'un membre de la famille de ligands TNF en tant que tel biologiquement inactif/actif d'une manière restreinte, sachant que le fragment dans la section (3) présente un domaine extracellulaire d'un membre de la famille de ligands TNF ou une variante de la séquence native du domaine extracellulaire homologue en séquence à raison d'au moins 80 %, et que le fragment dans la section (3) ne devient complètement actif du point de vue biologique sur la molécule de surface cellulaire que par liaison de la section (1).

2. Polypeptide selon la revendication 1, sachant que les sections (1) à (3) sont disposées de l'extrémité N-terminale à l'extrémité C-terminale.

3. Polypeptide selon la revendication 2, sachant que la section (3) contient les domaines extracellulaires de TRAIL, FasL, TNF, 41 BBL, CD40L, CD30L ou Ox4OL.

4. Polypeptide selon l'une quelconque des revendications précédentes, sachant que le domaine de liaison de molécule de surface cellulaire se lie à une protéine de membrane.

5. Polypeptide selon la revendication 4, sachant que le domaine de liaison de molécule de surface cellulaire comprend au moins la section d'un ligand d'un récepteur à membrane nécessaire à l'interaction spécifique avec la molécule de surface cellulaire.

6. Polypeptide selon la revendication 5, sachant que le ligand est une hormone de peptide ou de protéine liant un récepteur ou un fragment liant un récepteur d'une cytokine.

7. Polypeptide selon la revendication 6, sachant que le fragment de cytokine est une fragment d'un membre de la famille des ligands TNF, sous réserve que le membre de la famille des ligands TNF est différent de celui des sections (1) et (3).

8. Polypeptide selon la revendication 6, sachant que l'hormone est sélectionnée parmi le groupe se composant de facteurs de croissance, en particulier d'EGF, et de facteurs d'angiogenèse, en particulier de VEGF.

9. Polypeptide selon la revendication 4, sachant que le domaine de liaison de molécule de surface cellulaire comprend au moins la section d'un récepteur pour un ligand à membrane nécessaire à l'interaction spécifique avec la molécule de surface cellulaire, sous réserve que la section de récepteur ne se lie pas au domaine extracellulaire du membre de la famille des ligands TNF selon la section (3).

10. Polypeptide selon la revendication 9, sachant que le récepteur est choisi parmi la superfamille de récepteurs TNF.

11. Polypeptide selon la revendication 9 ou 10, sachant que le récepteur est choisi parmi le groupe se composant de TNF-R2, CD30, CD40 et CD28.

12. Produit d'assemblage d'acides nucléiques, contenant une séquence de nucléotides, qui code pour un polypeptide conformément à l'une des revendications précédentes.

13. Vecteur, contenant le produit d'assemblage d'acides nucléiques selon la revendication 12.

14. Cellule hôte, contenant le produit d'assemblage d'acide nucléiques selon la revendication 12 et/ou le vecteur selon la revendication 13.

15. Procédé d'isolement d'un polypeptide selon l'une des revendications 1 à 11, sachant que
(a) un vecteur selon la revendication 13 ou un produit d'assemblage d'acides nucléiques selon la revendication 12 est fabriqué,
(b) les cellules sont transfectées avec un vecteur ou un produit d'assemblage d'acides nucléiques obtenu conformément à l'étape de procédé (a),
(c) les cellules transfectées selon (b) sont cultivées et
(d) des polypeptides exprimés sous les conditions correspondantes sont isolés à partir des cellules hôtes et/ou du surnageant de culture.

16. Polypeptide selon l'une quelconque des revendications 1 à 11, produit d'assemblage selon la revendication 12, vecteur selon la revendication 13 ou cellule hôte selon la revendication 14 en tant que médicament.

17. Polypeptide selon l'une quelconque des revendications 1 à 11, produit d'assemblage d'acides nucléiques selon la revendication 12, vecteur selon la revendication 13 ou cellule hôte selon la revendication 14 pour utilisation dans le traitement de maladies cancéreuses, en particulier de tumeurs solides ou lymphatiques, de maladies infectieuses, de maladies du métabolisme, de troubles inflammatoires, de maladies auto-immunes, en particulier de maladies de polyarthrite rhumatoïde.

18. Composition pharmaceutique, contenant au moins un polypeptide selon l'une quelconque des revendications 1 à 11 et/ou un produit d'assemblage d'acides nucléiques selon la revendication 12 et/ou un vecteur selon la revendication 13 et/ou une cellule hôte selon la revendication 14 ainsi que des adjuvants, des additifs et/ou des excipients inoffensifs du point de vue physiologique.

19. Composition pharmaceutique selon la revendication 18 pour utilisation dans le traitement de maladies cancéreuses, en particulier de tumeurs solides ou lymphatiques, de maladies infectieuses, de maladies du métabolisme, de troubles inflammatoires, des maladies auto-immunes, en particulier de maladies de polyarthrite rhumatoïde.
